# EUROPEAN PATENT SPECIFICATION

(11) **EP 4 463 821 B1**
(45) Date of publication and mention of the grant of the patent: **29.04.2026**
(21) Application number: 23700137.5
(22) Date of filing: 10.01.2023
(51) Int. Cl.: G06T 7/10, A61B 8/08, G06T 7/00, A61B 8/00

(54) **SYSTEMS, METHODS, AND APPARATUSES FOR PLEURAL LINE DETECTION**
SYSTEME, VERFAHREN UND VORRICHTUNGEN ZUR ERFASSUNG VON PLEURALLINIEN
SYSTÈMES, PROCÉDÉS ET APPAREILS DE DÉTECTION DE LIGNE PLEURALE

(30) Priority: 12.01.2022 WO PCT/CN2022/071512; 14.04.2022 EP 22168408
(43) Date of publication of application: 20.11.2024
(60) Divisional application: 26150261.1
(73) Proprietor: Koninklijke Philips N.V., 5656 AG Eindhoven (NL)
(72) Inventor: XU, Jingping, 5656 AG Eindhoven (NL); RAJU, Balasundar Iyyavu, 5656 AG Eindhoven (NL); CHEN, Alvin, 5656 AG Eindhoven (NL)
(74) Representative: Philips Intellectual Property & Standards
(86) International application number: PCT/EP2023/050444
(87) International publication number: WO 2023/135128

(56) References cited:
- WO-A1-2015/048767
- WO-A1-2018/108742
- US-A1- 2019 105 013
- CHEN JIANGANG ET AL: "Automated Pleural Line Detection Based on Radon Transform Using Ultrasound", ULTRASONIC IMAGING., vol. 43, no. 1, 1 January 2021 (2021-01-01), US, pages 19 - 28, XP055962605, ISSN: 0161-7346, DOI: 10.1177/0161734620976408

## Description

### FIELD OF THE INVENTION

The present disclosure pertains to imaging systems and methods for automatically detecting a pleural line in an image. In particular, imaging systems and methods for automatically detecting a pleural line based on rib location in ultrasound images are disclosed.

### BACKGROUND OF THE INVENTION

Lung ultrasound may be useful in the management of a spectrum of respiratory diseases, particularly at the point of care. Its advantages include avoidance of patient transport to receive a computed tomography (CT) scan or chest x-ray thereby improving infection control procedures. There is growing interest in developing automated solutions for lung ultrasound to address clinical applications including the management of infectious diseases such as COVID-19, pneumonia, chronic pulmonary diseases, and chronic or congestive heart failure. In these applications, automated lung ultrasound would aid inexperienced users in triage and clinical decision-making when monitoring disease progression over time.

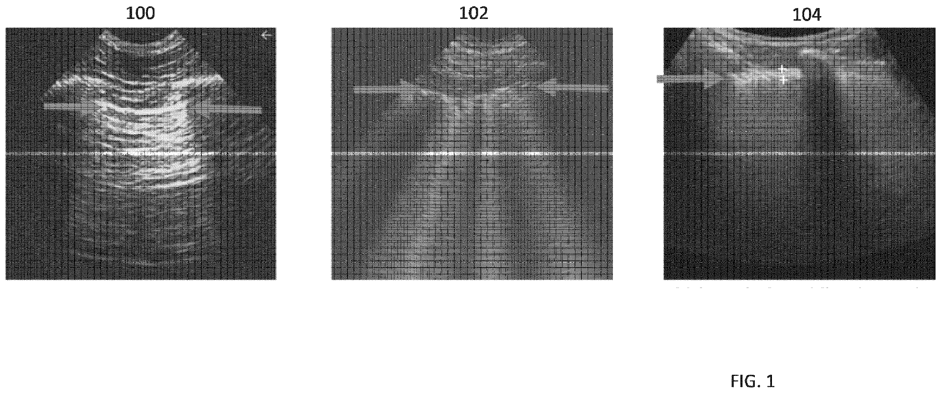

The pleural line is an important landmark in lung ultrasound. Other lung features such as B-lines and sub-pleural consolidations are related to the position of the pleural line. Recent clinical literature shows that changes in the pleural line, such as thickening or irregularity, are associated with the severity of respiratory and infectious diseases including COVID-19 pneumonia. FIG. 1 includes example ultrasound images including the pleural line. In images 100, 102, and 104, the pleural line is indicated by the arrows. Image 100 depicts a pleural line in a healthy patient. The pleural line is thin, less than or equal to approximately 2mm, and the pleural line appears smooth and continuous in the image 100. Image 102 depicts a pleural line in a patient with a respiratory disease such as pneumonia or acute respiratory disease. The pleural line appears irregular and interrupted within image 102. Image 104 depicts a pleural line in a patient with viral pneumonia, such as pneumonia associated with COVID-19. In image 104, the pleural line is thickened, greater than approximately 3mm, which may be due to subpleural fibrotic abnormalities. Thus, accurate detection of the pleural line may be an important first step for determining lung health.

Currently, manual identification and measurement of properties of the pleural line, such as by emergency physicians, is a time-consuming process and interpretation of the ultrasound images and evaluation of the pleural line is subjective. Previous approaches for automatic or semi-automatic pleural line detection have used a variety of techniques including confidence map-based approach, 3-seed points based peak detection, or deep-learning based approach. However, each of these previous methods have limitations. In the confidence-map based approach, the region with values bigger than the global threshold of entire map may be considered the upper pleura and the remaining may be considered the lower pleural region. Confidence maps may provide reliable pleural line detection from the sharp drop of confidence value along axial direction. However, radio-frequency ultrasound signal is needed for computing confidence map with high computation load which may not be available in most portable point-of-care (POC) ultrasound imaging systems. The 3-seed point based peak detection approach may work well for most cases, but may suffer from false-positives for cases where there are 1) forced breathing from the patient in both horizontal and vertical directions and 2) strong motion around location of A-line.

A deep learning based pleural line detection approach using a multi-layer convolutional neural network (CNNs) with collected ultrasound sequence frames as input may be used. The prediction model is trained by a relative small amount of lung ultrasound images. The major limitation for deep learning-based approach is requirements for large dataset from multiple clinical sites and significant annotation of the images. Furthermore, lack of transparency of detected features from deep learning is still an issue for clinical acceptance and regulatory approval.

US 2019/105013 A1 discloses a method for estimating the depth of the pleural line by deriving an intensity profile representing an intensity value as a function of depth, each intensity value indicating an averaged ultrasound data value at the corresponding depth; the depth of the pleural interface is estimated as the depth of the peak of the intensity profiles.

In "Automated Pleural Line Detection Based on Radon Transform Using Ultrasound" by Chen Jiangang et al., Ultrasonic Imaging, Vol. 43, No. 1, pp. 19-28, 1 January 2021 (XP055962605), United States, it is presented a computer-aided technique for automated pleural line detection using ultrasound; it uses the Radon transform to detect line objects in the ultrasound images.

WO 2018/108742 A1 discloses a method for identification of one or more candidate pleural lines; it includes creating an intensity profile that represents intensity values as a function of depth at a number of lateral positions encompassed by an ultrasound scan.

Accordingly, improved automatic pleural line detection techniques are desired.

### SUMMARY OF THE INVENTION

The invention is defined by the independent claims. Advantageous embodiments are provided in the dependent claims.

A method and system for automated pleural line detection in lung ultrasound images may include selecting a region of interest (ROI) in image data (the first ROI image) that is likely to encompass the rib shadow, typical ROI image depth of 2.5 cm to 3 cm starting from the bottom of the image (e.g., range of [(depth-2.5) to depth]), summing intensity along the axial direction to obtain an intensity projection curve for each scanning line on the first ROI image, determining the small value regions in the intensity projection curve which could be rib candidates due to the small value regions are from rib shadows, determining the second ROI image from the small value region, summing intensity along the lateral direction to obtain an axial intensity projection curve on the second ROI image, determining the surface of the rib by reversing searching peaks from bottom-up on the axial intensity projection curve, determining the third ROI image starting from the surface of the rib and 1 cm deeper, computing lung sliding motion map from several adjacent frames (e.g., 9 to 16 frames) on the region of the third ROI image, using the locations of rib to limiting searching region on lung sliding motion map where the largest motion increase is from the pleural line, displaying the detected pleural line using a color indicator on the screen. The techniques disclosed herein may also be used to detect ribs and/or surfaces of ribs and display visual indicators thereof.

The system and methods disclosed herein may provide a uniform method that all operators can follow, thereby reducing operator dependence.

According to at least one example of the present disclosure, a method for determining a pleural line in an image may include automatically selecting a first region of interest (ROI) within an image acquired from a lung of a subject based, at least in part, on a depth of the image, analyzing the first ROI to determine at least one rib shadow region in the image, based, at least in part, on the rib shadow region, automatically selecting a second ROI within the image, analyzing the second ROI to determine a location of a rib surface in the image, based, at least in part, on the location of the rib surface, automatically selecting a third ROI within the image, analyzing the third ROI, and determining the pleural line based on the analyzing of the third ROI.

In some examples, the first ROI extends from a pre-determined depth to the depth of the image.

In some examples, analyzing the first ROI comprises computing, with at least one processor, a lateral projection for the first region of interest.

In some examples, the second ROI extends from a top of the image to the depth of the image and extends across a portion of a width of the image based on a comparison of lateral intensities of the lateral projection to a threshold value.

In some examples, analyzing the second ROI includes computing, with at least one processor, an axial projection for the second region of interest, and detecting, with the at least one processor, a peak in the axial projection.

In some examples, the third ROI extends from a first depth in the image corresponding to a location of the peak to a second depth greater than the first depth.

In some examples, the peak is detected based, at least in part, on a comparison of a difference between a peak value and a neighboring value and a threshold value.

In some examples, analyzing the third ROI includes computing, with at least one processor, an axial projection for the third region of interest, detecting, with the at least one processor, one or more peaks in the axial projection, wherein individual ones of the one or more peaks correspond to a corresponding candidate pleural line, and computing, with the at least one processor, a motion map for the third region of interest.

In some examples, determining the pleural line includes, for individual ones of the corresponding candidate pleural lines: calculating an average motion above the candidate pleural line and an average motion below the candidate pleural line from the motion map, and calculating a difference between the average motion above and the average motion below the candidate pleural line, determining the candidate pleural line having a greatest difference between the average motion above and the average motion below, and selecting the candidate pleural line having the greatest difference as the pleural line. In some examples, the method may further include determining a location of individual ones of the candidate pleural lines based, at least in part, on locations of corresponding ones of the one or more peaks. In some examples, the method may further include determining a local brightness of individual ones of the candidate pleural lines.

In some examples, the method may include analyzing the pleural line to determine a width, a thickness, a smoothness, or a combination thereof, of the pleural line.

In some examples the method may include displaying the image on a display with a visual indicator of the pleural line overlaid on the image.

According to at least one example of the present disclosure, an ultrasound imaging system configured to determine a pleural line in an ultrasound image may include an ultrasound probe configured to acquire an ultrasound image from a lung of a subject, at least one processor configured to automatically select a first region of interest (ROI) within the ultrasound image based, at least in part, on a depth of the ultrasound image, analyze the first ROI to determine at least one rib shadow region in the ultrasound image, based, at least in part, on the rib shadow region, automatically select a second ROI within the ultrasound image, analyze the second ROI to determine a location of a rib surface in the ultrasound image, based, at least in part, on the location of the rib surface, automatically select a third ROI within the ultrasound image, analyze the third ROI, and determine the pleural line based on the analyzing of the third ROI, and a display configured to display a visual indication of the pleural line overlaid on the ultrasound image.

In some examples, the system may include a user interface configured to receive a user input indicating an age, a weight, or a combination thereof, of the subject, wherein the user input determines, at least in part, the first ROI.

According to at least one example of the present disclosure, a method for determining a rib surface in an image may include automatically selecting a first region of interest (ROI) within an image acquired from a lung of a subject based, at least in part, on a depth of the image, analyzing the first ROI to determine at least one rib shadow region in the image, based, at least in part, on the rib shadow region, automatically selecting a second ROI within the image, and analyzing the second ROI to determine a location of a rib surface in the image.

In some examples, analyzing the second ROI includes computing, with at least one processor, an axial projection for the second region of interest and detecting, with the at least one processor, a peak in the axial projection.

In some examples, the peak is detected based, at least in part, on a comparison of a difference between a peak value and a neighboring value and a threshold value.

In some examples, the method may further include displaying the image on a display with a visual indicator of the rib surface overlaid on the image.

In some examples, the first ROI extends from a pre-determined depth to the depth of the image, analyzing the first ROI comprises computing, with at least one processor, a lateral projection for the first region of interest, and the second ROI extends from a top of the image to the depth of the image and extends across a portion of a width of the image based on a comparison of lateral intensities of the lateral projection to a threshold value.

### BRIEF DESCRIPTION OF THE DRAWINGS

Fig. 1 includes example ultrasound images including the pleural line.
Fig. 2 provides two illustrations depicting ultrasound probe placement during a lung ultrasound scan.
Fig. 3 shows example ultrasound images from a lung ultrasound scan.
Fig. 4 is a flow chart summarizing a technique for detecting a pleural line in accordance with examples of the present disclosure.
Fig. 5A is a graphic illustrating how a lateral projection is computed in accordance with examples of the present disclosure.
Fig. 5B is a graphic illustrating how an axial projection is computed in accordance with examples of the present disclosure.
Fig. 6 provides an illustration of relationships between an ultrasound image and regions of interest in accordance with examples of the present disclosure.
Fig. 7A is an example of an ultrasound image of a lung from a subject with COVID-19 pneumonia.
Fig. 7B is a plot of an example of a lateral projection profile computed in accordance with examples of the present disclosure.
Fig. 8A is a second region of interest of the ultrasound image shown in Fig. 7A in accordance with examples of the present disclosure.
Fig. 8B is a plot of an example of an axial projection profile computed in accordance with examples of the present disclosure.
Fig. 9A is a color map version of the ultrasound image shown in Fig. 7A.
Fig. 9B is an example motion map computed for the third region of interest in accordance with examples of the present disclosure.
Fig. 10 shows the ultrasound image shown in Fig. 7A with a visual indication of the pleural line in accordance with examples of the present disclosure.
Fig. 11 is a block diagram of an ultrasound system in accordance with examples of the present disclosure.
Fig. 12 is a block diagram illustrating an example processor in accordance with principles of the present disclosure.
Fig. 13 is a flow chart of a method in accordance with examples of the present disclosure.
Fig. 14 shows the ultrasound image shown in Fig. 7A with a visual indication of a rib surface in accordance with examples of the present disclosure.

### DETAILED DESCRIPTION OF EMBODIMENTS

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. Moreover, for the purpose of clarity, detailed descriptions of certain features will not be discussed when they would be apparent to those with skill in the art so as not to obscure the description of the present apparatuses, systems, and methods. The following detailed description is therefore not to be taken in a limiting sense, and the scope of the present system is defined only by the appended claims.

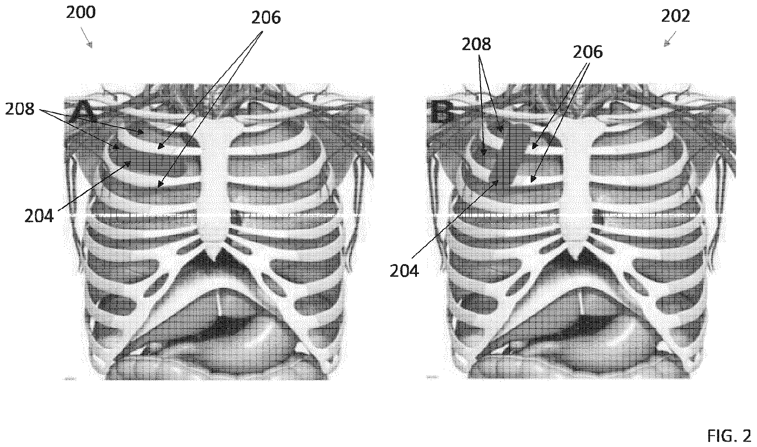

Fig. 2 provides two illustrations depicting ultrasound probe placement during a lung ultrasound scan. Both illustrations 200 and 202 include an oval shape 204 indicating placement of an ultrasound probe relative to ribs 206 and intercostal spaces 208. During a lung ultrasound scan, an ultrasound probe is typically placed longitudinally to the ribs 206 over the intercostal space 208 as indicated by the oval shape 204 depicted in illustration 202.

Ultrasound signals (e.g., beams) propagate through skin, subcutaneous tissue and muscle (e.g., chest wall soft tissue) to reach a surface of a rib and a visceral pleural-lung interface. At the rib surface, the ultrasound signals are almost totally reflected back (e.g., echo signals) and received by the ultrasound probe due to large acoustic impedance between the bony surface and its nearby soft tissue. The rib in the resulting ultrasound images may appear as a convex hyperechoic bow-like line. Regions of the lung "behind" the rib relative to the ultrasound probe may receive little to no ultrasound signals due to the high reflection of the ribs. Thus, in the resulting ultrasound images, these regions may appear as hypoechoic regions (e.g., lower amplitude, darker regions) which may extend from the rib to the bottom of the ultrasound image. These regions may be referred to as rib shadows.

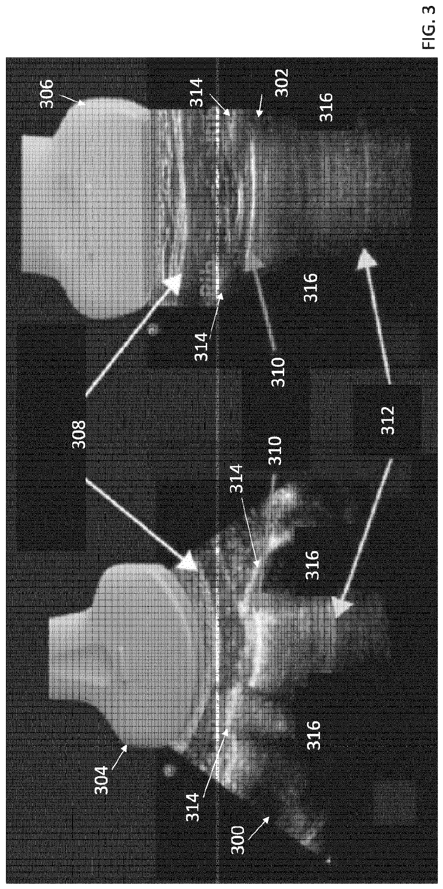

Fig. 3 shows example ultrasound images from a lung ultrasound scan. Image 300 was acquired with a curved (e.g., sector) probe 304. Image 302 was acquired with a linear probe 306. In both image 300 and image 302, the subcutaneous tissues and intercostal muscles 308, pleural line 310, A-lines 312, ribs 314, and rib shadows 316 can be observed. While both images 300, 302 show two ribs 314, some images acquired during an ultrasound scan may only include one rib, typically on a left-side of the image relative to a view of the image. Whether one or two ribs are present and its location may be based, at least in part, on a relative size of the probe to a subject being scanned, a position of the probe, an orientation of the probe, and/or imaging protocols in place where the lung ultrasound scan is being conducted.

The pleural line (e.g., pleural lines 310) is usually a horizontal hyperechoic line approximately 0.5 cm deeper than the rib surface in adults. The inventors have recognized that as long as the surface of the rib can be determined, then a searching region for the pleural line can be selected to be 0.5~1.0 cm deeper than the location of the rib. Defining this searching region may reduce the possible false positives of the pleural line from superficial horizontal bright lines which are usually located at the depth range of 0.6 cm to 3 cm, and this range is overlapped with location of pleural lines for some subjects, strong intensity from A-lines, especially in cases where the pleural line is below or around 1 cm, and bright horizontal lines at superficial region (e.g., intercostal muscles 308).

According to examples of the present disclosure, a first region of interest (ROI) is automatically selected within an ultrasound image acquired from a lung ultrasound scan of a patient based, at least in part, on a depth of the image. The first ROI may be analyzed to determine at least one rib shadow region. In some examples, a lateral projection is computed for the first ROI for analysis. The rib shadow region may be used to automatically select a second ROI. The second ROI may be analyzed to determine a location of a rib surface. In some examples, the second ROI may be used to compute an axial projection for analysis. The location of the rib surface may be used to automatically select a third ROI. The third ROI may be analyzed to identify the pleural line. In some examples, the third ROI may be used to compute an axial projection and/or a motion map for analysis. Using the systems and methods disclosed herein, the pleural line may be correctly identified (e.g., a depth/location of the pleural line may be determined) based on the position of the rib surface and rib shadows in the lung ultrasound image, even in difficult cases.

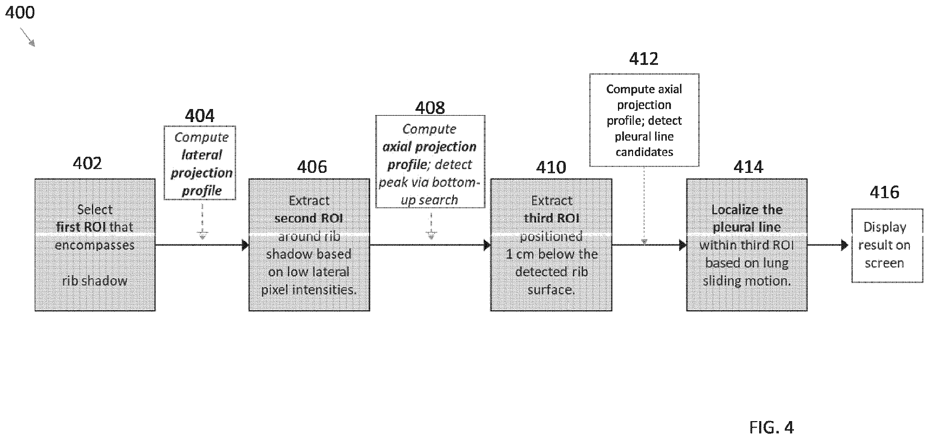

Fig. 4 is a flow chart summarizing a technique for detecting a pleural line in accordance with examples of the present disclosure. In some examples, the technique shown in flow chart 400 may be performed in whole or in part by an ultrasound imaging system. In some examples, the technique shown in flow chart 400 may be performed at least in part, by one or more processors, which may be included in an ultrasound imaging system and/or in a computing system used for post-processing of ultrasound images. The technique shown in flow chart 400 may be performed on an ultrasound image acquired from a lung of a subject. The ultrasound image may include at least one rib, a rib shadow, and a pleural line. For example, the ultrasound images 100, 102, and 104 of Fig. 1 and/or ultrasound images 300 and 302 of Fig. 3.

As indicated by block 402, within an ultrasound image acquired from a lung of a subject, a first ROI may be selected. The first ROI may extend from a pre-determined depth as measured from a face of the ultrasound probe to the bottom of the image across an entire width of the image. Alternatively the pre-determined depth may be defined relative to the bottom of the image.

The pre-determined depth may be approximately 4 cm to 5 cm for a typical adult subject (e.g., alternatively, 2 cm-3 cm from the bottom of the image). In some examples, the pre-determined depth may be adjusted based, at least in part, on the type of ultrasound probe used, whether the subject is an adult or a child, whether or not the subject is overweight. For example, if the subject is a child, the pre-determined may be less (e.g., approximately 2 cm to 3 cm). In another example, if the subject is an overweight adult, the pre-determined depth may be greater (e.g., approximately 5-5.5 cm). In some examples, the pre-determined depth may be adjusted (if necessary) automatically, for example, an ultrasound imaging system may detect a type of ultrasound probe being used and/or an ultrasound image may have metadata (e.g., DICOM info head/tag) indicating the type of probe used that can be interpreted by a computing system. In some examples, the pre-determined depth may be adjusted based on input from a user (e.g., emergency physician, ultrasound technician). For example, during the ultrasound exam, a user interface of the ultrasound system may prompt the user for information about the subject and adjust the pre-determined depth (if necessary) based on the user's input.

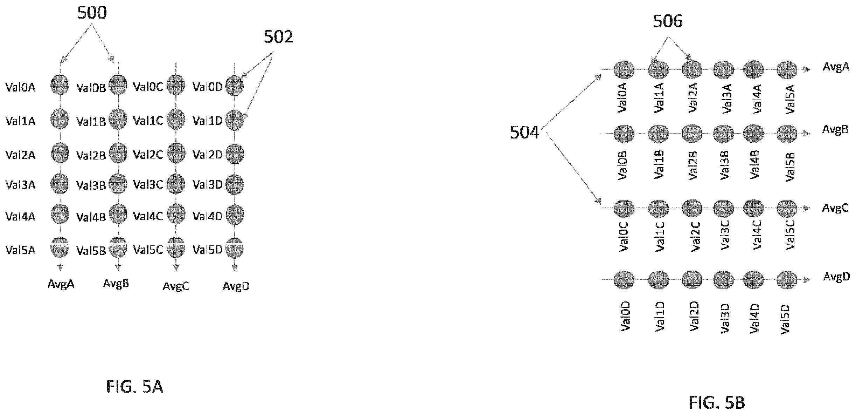

Within the first ROI, a lateral projection is computed as indicated by block 404. Fig. 5A is a graphic illustrating how a lateral projection is computed in accordance with examples of the present disclosure. For each scan line 500 of an ultrasound image, the intensity values Val0-5A-D of all the pixel 502 along the scan line 500 are averaged. The average values AvgA-D may then be plotted as a curve along the lateral direction (e.g., perpendicular to the scan lines) to provide the projection. The average values may be referred to as lateral intensities.

Returning to Fig. 4, a second ROI may be selected within the ultrasound image based on the lateral projection as indicated by block 406. Pixels of scan lines having lower average values may be associated with regions of the image that include the rib shadow and pixels of scan lines having higher average values may be associated with regions of the image that include lung and other tissue that are not shadowed by the ribs. The second ROI may be selected to include one or more regions of the ultrasound image corresponding to lateral intensities equal to and/or below a threshold value (e.g., equal to or below a value of 6 on an intensity scale of 0-255).

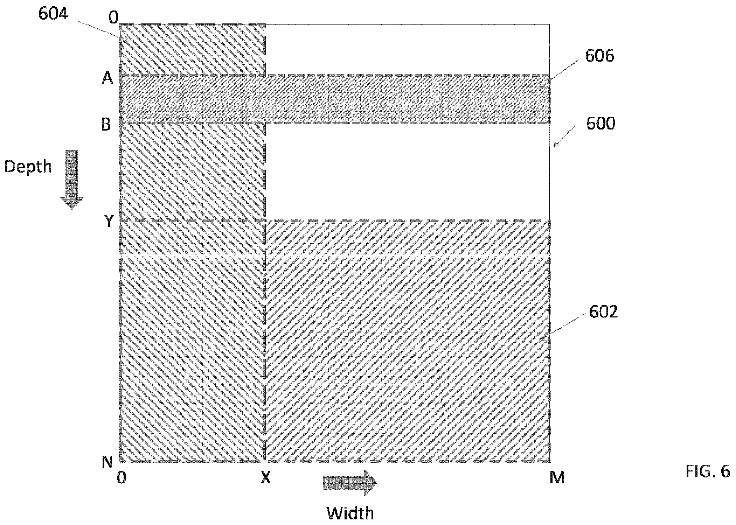

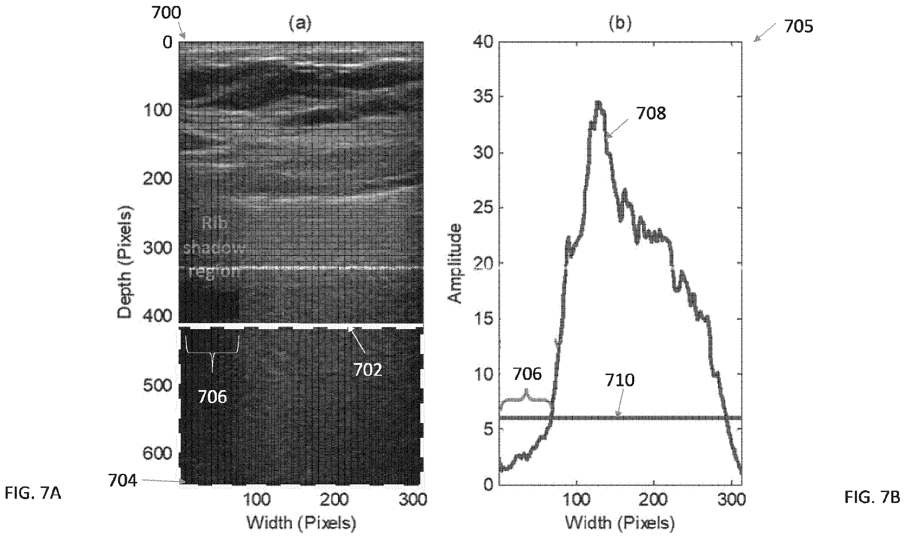

Fig. 6 provides an illustration of relationships between an ultrasound image and regions of interest in accordance with examples of the present disclosure. Block 600 represents an ultrasound image, block 602 represents the first ROI, and block 604 represents the second ROI. Block 606 represents a third ROI, which will be described in more detail below. The ultrasound image 600 may have a depth of 0-N pixels and a width of 0-M pixels. The pixels may correspond to spatial locations within a subject scanned by an imaging plane corresponding to the ultrasound image 600. The 0 pixel in the depth direction corresponds to a spatial location most proximate to the ultrasound probe and the N pixel in the depth direction is the pixel most distal from the ultrasound probe. In some examples, the first ROI 602 may extend for an entire width of the ultrasound image 600 (e.g., pixel 0 to pixel M), and extend from a pre-determined depth (e.g., pixel Y) to the bottom of the ultrasound image 600 (e.g., pixel N). In some examples, the pre-determined depth may be below the top of the ultrasound image 600 (e.g., a pixel greater than 0). In some examples, the second ROI 602 may extend an entire depth of the ultrasound image 600 (e.g., pixel 0 to pixel N), but only include a width (or widths if two ribs are present) of the ultrasound image 600 corresponding to the region with the lateral intensities below the threshold value (e.g., pixel 0 to pixel X). In some examples, the width of the second ROI 604 may be less than a width of the ultrasound image 600 (e.g., less than M pixels wide). An example of finding the first and second ROIs in an ultrasound image is shown in Figs. 7A and 7B.

Fig. 7A is an example of an ultrasound image of a lung from a subject with COVID-19 pneumonia. The image 700 is an image from a sequence of two dimensional (2D) images acquired by a Philips Lumify ultrasound imaging system with a linear probe of L12-4 at a rate of approximately 30 Hz with a maximum depth of approximately 7 cm. The first ROI 704 within the image 700 is indicated by the dashed box. In some examples, the first ROI 704 may be automatically selected based on a pre-determined depth 702. In the example shown in Fig. 7A, the pre-determined depth 702 is 2.5 cm from the bottom of the image 700 (e.g., a depth of 4.5 cm).

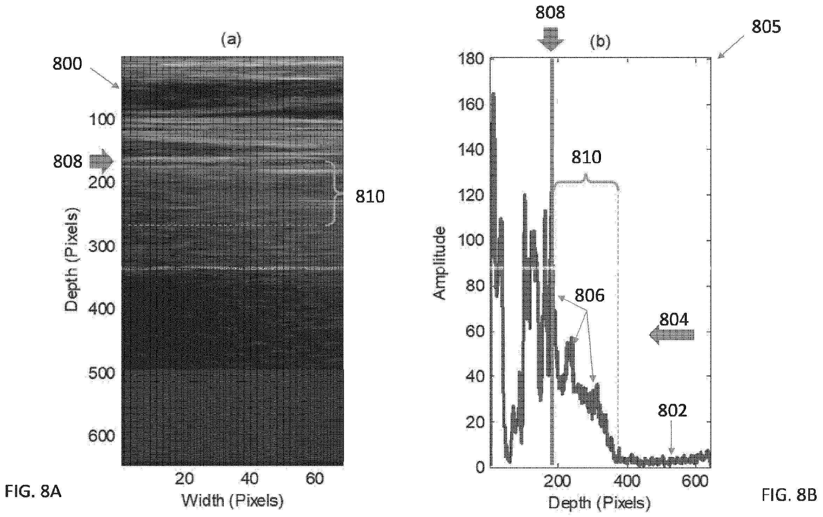

Fig. 7B is a plot of an example of a lateral projection profile computed in accordance with examples of the present disclosure. The plot 705 shows a curve of the amplitudes of the lateral intensities 708 plotted over the width of the ultrasound image 700. Pixels associated with lateral intensities 708 equal to and/or below a threshold value, indicated by line 710, may be determined to correspond to a rib shadow region 706. In the example shown in Fig. 7B, the threshold value is 6, pixels 0-68 in the width direction are determined to correspond to the rib shadow region. The second ROI is then selected to be the entire depth of the ultrasound image and extend for a width of 68 pixels. The second ROI 800 from image 700 is shown in Fig. 8A.

Continuing with the example shown in Fig. 7B, the lateral intensities 708 for pixels 293-312 are also equal to and/or below the threshold value. These pixels may correspond to another rib shadow region due to another rib. Because the rib shadow region from pixels 0-68 is larger, only that region may be selected for the second ROI. In some examples, such as in the present example, only one rib shadow region may be selected as the second ROI. In some examples, if two rib shadow regions are determined, the wider of the two rib shadow regions is selected. In some examples, if two rib shadow regions are determined, only the region to the left-most or right-most region of the ultrasound image 700 is selected. In some examples, if two rib shadow regions are determined, only one region is selected as the second ROI if one of the regions is below a threshold width (e.g., less than 30 pixels).

Returning to Fig. 4, within the second ROI, an axial projection is computed to determine a location of a rib surface as indicated by block 408. Fig. 5B is a graphic illustrating how an axial projection is computed in accordance with examples of the present disclosure. For each depth 504 of an ultrasound image, the intensity values Val0-5A-D of all the pixel 506 for a given depth 504 are averaged. The average values AvgA-D may then be plotted as a curve along the axial direction (e.g., parallel to the scan lines) to provide the axial projection. The average values may be referred to as axial intensities. The curve of axial intensities may be analyzed to detect peaks starting at the bottom (e.g., greatest depth) of the image. Any known or future known peak detection technique may be used. Optionally, in some examples, the curve may be smoothed prior to peak detection. When a peak meets and/or exceeds a threshold value, a rib surface may be located at the depth associated with the higher average axial intensity value. In some examples, the first peak (e.g., peak with the greatest depth) to meet and/or exceed the threshold value may be determined to be the rib surface. In examples where two rib shadow regions are detected and selected as second ROIs, the technique may be performed twice to find a rib surface in each of the second ROIs.

Because the rib shadow found in the first ROI was used to select the second ROI, and the rib shadow is caused by the rib, in some applications, the probability of finding the rib within the second ROI may be higher than other techniques for finding the rib surface that do not rely on the location of the rib shadow region. Furthermore, since the peak detection is performed starting at a greater depth and working towards shallower depths, there may be fewer false positives due to peaks caused by strong reflectors in the subcutaneous tissues and intercostal muscles.

A third ROI may be selected within the ultrasound image based on the determined depth of the rib surface as indicated by block 410. In some examples, the third ROI may extend from the determined depth of the rib surface to a pre-determined depth below the rib surface. In typical adults, the pleural line is around 0.5 cm below the rib surface. Accordingly, in some examples, the pre-determined depth may be 1 cm. This may provide a margin for biological variability while still limiting the region in which to search for the pleural line. Limiting the search region may reduce computing time and/or reduce false positives in some applications. In some examples, the third ROI may further extend to a depth slightly above the rib surface (e.g., 0.2 cm). The relationship of the third ROI to the ultrasound image, the first ROI, and the second ROI is illustrated in Fig. 6 where block 606 represents the third ROI. In some examples, the third ROI 606 may extend an entire width of the image 600 (e.g., pixel 0 to pixel M) and extend from a first depth (e.g., pixel A) to a second depth (e.g., pixel B). In some examples, the first depth may correspond to a location of the peak found in the axial projection in the second ROI (e.g., the determined rib surface), and the second depth (which may be deeper than the first depth) may correspond to the pre-determined depth below the rib surface. An example of finding the third ROI in the ultrasound image from the second ROI is shown in Figs. 8A and 8B.

Fig. 8A is a second region of interest of the ultrasound image shown in Fig. 7A in accordance with examples of the present disclosure. The second ROI 800 extends the full depth of the ultrasound image 700, but the width of the second ROI 800 is based on the determined rib shadow region 706 computed from the lateral projection plot 705. In the example shown in Fig. 8A, the width of the second ROI 800 is 68 pixels.

Fig. 8B is a plot of an example of an axial projection profile computed in accordance with examples of the present disclosure. The plot 805 shows a curve of the amplitudes of the axial intensities 802 plotted over the depth of the ultrasound image 700. The curve 802 is analyzed beginning at the bottom of the ultrasound image 700 (e.g., highest pixel number, greatest depth) and moving towards the top (e.g., lower pixel number, lesser depth) as indicated by arrow 804 using peak detection to detect local peaks 806. Individual detected peaks 806 may be analyzed to determine the height of the peak 806 relative to the surrounding values on either side of the peak 806 along the curve 802. In some examples, the height of the peak 806 may be compared to an average value of the surrounding values. If the height of the peak 806 is less than and/or equal to a threshold value, the peak 806 may be rejected as a location (e.g., depth) of a rib surface and the next peak 806 along the curve 802 in the direction indicated by arrow 804 may be analyzed. When the height of the peak 806 is greater than and/or equal to the threshold value, the location of the peak 806 may be determined to be a location of the rib surface. In the example shown in Fig.8B, the location of the rib surface 808 is at pixel 180 as indicated by the vertical line.

As shown in Fig. 8A, the third ROI 810 is selected to be from the location of the rib surface 808 to a depth of 1 cm below the rib surface 808. In the example shown in Fig. 8A, the third ROI 810 extends from pixel 180 to pixel 271. It is noted that Fig. 8A only shows a portion of the third ROI 810. While the full depth of the third ROI 810 is shown, the third ROI 810 extends across the entire width of ultrasound image 700.

Returning to Fig. 4, within the third ROI, an axial projection may be computed as indicated by block 412. The axial projection may be computed using similar techniques as used to compute the axial projection for the second ROI as described with reference to Figs. 5B, 8A, and 8B. The resulting curve of axial intensities may be analyzed to detect local peaks. Any known or future known peak detection technique may be used. Optionally, similar to the peak detection in the second ROI, smoothing of the curve of axial intensities may be performed prior to peak detection. In some examples, all local peaks may be determined to be candidate pleural lines. In some examples, local peaks having a difference in intensity relative to surrounding values equal to and/or greater than a threshold value may be determined to be candidate pleural lines.

Within the third ROI, a motion map is generated based from a series of ultrasound images. The series ultrasound images may include the ultrasound image used to select the ROI. The ultrasound image may be one frame in a multi-frame image (e.g., a cine-loop, movie) all acquired by an ultrasound probe at a same (or approximately same) location and orientation at different times. The series of ultrasound images used to generate the motion map may be frames temporally adjacent to one another. In some examples, the ultrasound image may be the first frame, the last frame, or a middle frame of the series of frames used to generate the motion map. In some examples, nine to sixteen adjacent frames may be used to generate the motion map. In other examples, more or fewer frames may be used.

For each pair of adjacent frames, in the third ROI, the magnitude of intensity differences between corresponding pixels are calculated. Differences in pixel intensity between adjacent frames may correlate to motion between the frames. The magnitudes of the differences for individual pixels are summed across all of the frames to compute a total magnitude value. Thus, each pixel in the ultrasound image may be associated with a value of total magnitude. Similar to pixels being assigned a color or grayscale value based on the intensity value associated with the pixel in a B-mode image, a color or grayscale value may be assigned to each pixel based on the value of total magnitude associated with the pixel. This color or grayscale mapping may be used to generate a motion map. Regions of greater motion may be associated with larger total magnitude values than regions of lesser motion. In some examples, even though the values of the motion map are calculated, the motion map may not be provided on a display.

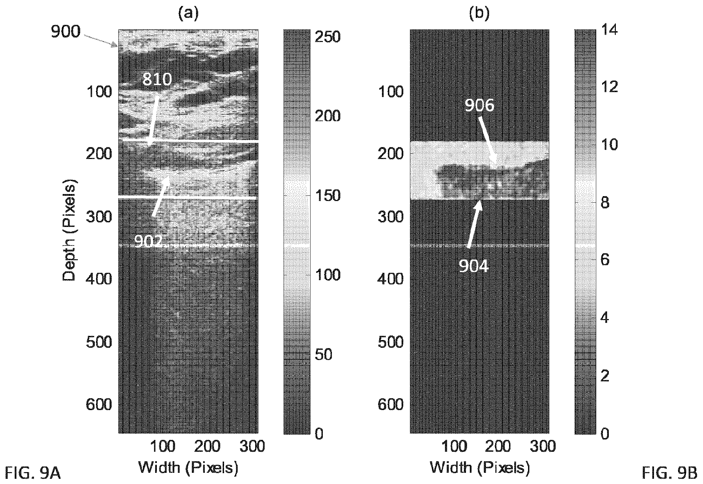

Fig. 9A is a color map version of the ultrasound image shown in Fig. 7A. The ROI 810 is indicated by the two parallel lines across the width of the color map version 900 of the ultrasound image 700. Within the third ROI, at least one candidate pleural line 902 is present. Fig. 9B is an example motion map computed for the third region of interest in accordance with examples of the present disclosure. In motion map 904, around depth indicated by arrow 906, there is a significant change in the magnitude of the motion. There is little to no motion above the depth indicated by arrow 906 and significant motion below the depth indicated by arrow 906. This may indicate a location of the pleural line. Typically, motion of the lung below the pleura is significant (e.g. due to lungs expanding and contracting with breathing) whereas there is little to no motion in the superficial tissue above the pleura. Thus, typically, there will be a significant difference between the motion above the pleural line and the motion below the pleural line.

As indicated by block 414 of Fig. 4, the location of the pleural line may be determined, at least in part, based on motion, such as lung sliding motion, based on the motion map. For each candidate pleural line determined based on peak detection of the axial projection of the third ROI, the average motion just above the candidate pleural line and the average motion below the pleural line are calculated based on the values in the motion map. In some examples, the candidate pleural line with the greatest average motion difference may be determined to be the "true" pleural line.

In some applications, using motion to determine the pleural line from multiple candidate pleural lines may allow detection of the pleural line when it is not the brightest line in the region. For example, fibers may be present in the vicinity of the pleural line in some lung conditions. Depending on the orientation and/or other properties, the fibers may reflect ultrasound signal more strongly than the pleural line. Thus, fibers may cause false positives in some cases when brightness is the sole criteria for assessing candidate pleural lines.

Optionally, in some examples, additional criteria may be used to determine the pleural line from multiple candidate pleural lines. For example, as previously noted, in adults, the pleural line is typically around 0.5 cm below the surface of the rib. In some examples, candidate pleural lines that are closer to this depth may be favored and/or selected over pleural lines further away from this depth. In some examples, the local brightness of the candidate pleural line may be used (e.g., the height of the peak relative to its surrounding values on the axial projection curve). Pleural lines with greater local brightness may be favored and/or selected over pleural lines with lower local brightness.

In some applications, one or more of the additional criteria may be required to determine the pleural line from candidate pleural lines. While the difference in motion above and below the pleural line may typically be a reliable indicator of the "true" pleural line, little to no lung sliding motion may be present in certain conditions. For example, in pneumothorax (PTX), lung sliding may not be present. When little to no motion is detected in the motion map, the pleural line may be determined based on depth and/or local brightness. In some examples, if no candidate pleural lines are found near the depth and/or have a local brightness equal to and/or above a threshold value, it may indicate that the pleural line cannot be determined from the candidate pleural lines. In some examples, the depth and/or local brightness may be used to eliminate some or all of the candidate pleural lines even when motion is present.

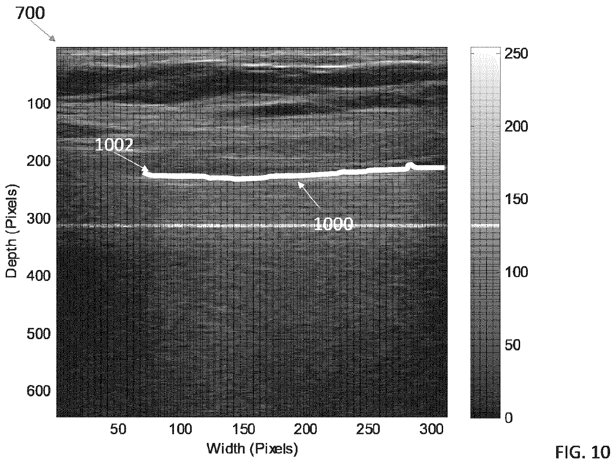

As described in block 416 of Fig. 4, the result of determining the pleural line in the ultrasound image may be displayed on a screen (or other display device). In some examples, the pleural line may be indicated by a visual indicator (e.g., highlighting, arrows, etc.) overlaid on the image. Fig. 10 shows the ultrasound image shown in Fig. 7A with a visual indication of the pleural line in accordance with examples of the present disclosure. In image 700, the pleural line 1000 is highlighted by a curve 1002. If the pleural line could not be determined, an indication of such may be provided (e.g., error text or symbol). In some examples, if the pleural line is determined, but no motion is detected, an indication of the lack of motion and/or possible existence of PTX in the subject may be provided on the screen.

Optionally, once the pleural line has been determined using the technique summarized in Fig. 4, the pleural line may be analyzed to determine one or more properties of the pleural line (e.g., pleural line 1000 in Fig. 10). For example, now known or future known segmentation techniques may be applied to the ultrasound image to segment the pleural line. The segmented image may be used to determine the position, width, thickness, and/or smoothness of the pleural line.

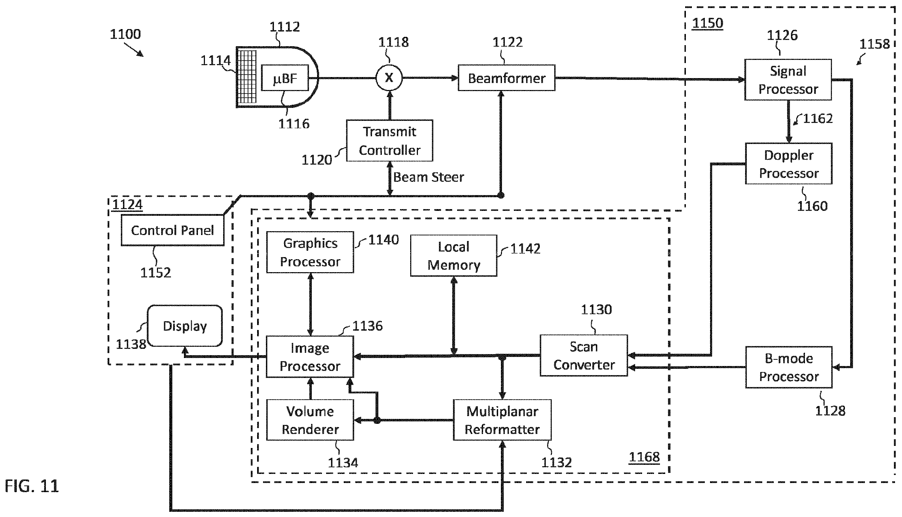

Fig. 11 shows a block diagram of an ultrasound imaging system constructed in accordance with examples of the present disclosure. In some examples, the ultrasound imaging system 1100 may determine a pleural line in an ultrasound image in accordance with examples of the present disclosure as described with reference to Figs. 4-10. The ultrasound imaging system 1100 may include a transducer array 1114, which may be included in an ultrasound probe 1112. In some examples, ultrasound probe 1112 may include ultrasound probe 304 and/or ultrasound probe 306. The transducer array 1114 is configured to transmit ultrasound signals (e.g., beams, waves) and receive echoes responsive to the ultrasound signals. A variety of transducer arrays may be used, e.g., linear arrays, curved arrays, or phased arrays. The transducer array 1114, for example, can include a two dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some embodiments, the transducer array 1114 may be coupled to a microbeamformer 1116, which may be located in the ultrasound probe 1112, and which may control the transmission and reception of signals by the transducer elements in the array 1114. In some embodiments, the microbeamformer 1116 may control the transmission and reception of signals by active elements in the array 1114 (e.g., an active subset of elements of the array that define the active aperture at any given time).

In some embodiments, the microbeamformer 1116 may be coupled, e.g., by a probe cable or wirelessly, to a transmit/receive (T/R) switch 1118, which switches between transmission and reception and protects the main beamformer 1122 from high energy transmit signals. In some embodiments, for example in portable ultrasound systems, the T/R switch 1118 and other elements in the system can be included in the ultrasound probe 1112 rather than in the ultrasound system base, which may house the image processing electronics. An ultrasound system base typically includes software and hardware components including circuitry for signal processing and image data generation as well as executable instructions for providing a user interface (e.g., processing circuitry 1150 and user interface 1124).

The transmission of ultrasonic signals from the transducer array 1114 under control of the microbeamformer 1116 is directed by the transmit controller 1120, which may be coupled to the T/R switch 1118 and a main beamformer 1122. The transmit controller 1120 may control the direction in which beams are steered. Beams may be steered straight ahead from (orthogonal to) the transducer array 1114, or at different angles for a wider field of view. The transmit controller 1120 may also be coupled to a user interface 1124 and receive input from the user's operation of a user control. The user interface 1124 may include one or more input devices such as a control panel 1152, which may include one or more mechanical controls (e.g., buttons, encoders, etc.), touch sensitive controls (e.g., a trackpad, a touchscreen, or the like), and/or other known input devices.

In some embodiments, the partially beamformed signals produced by the microbeamformer 1116 may be coupled to a main beamformer 1122 where partially beamformed signals from individual patches of transducer elements may be combined into a fully beamformed signal. In some embodiments, microbeamformer 1116 is omitted, and the transducer array 1114 is under the control of the main beamformer 1122 which performs all beamforming of signals. In embodiments with and without the microbeamformer 1116, the beamformed signals of the main beamformer 1122 are coupled to processing circuitry 1150, which may include one or more processors (e.g., a signal processor 1126, a B-mode processor 1128, a Doppler processor 1160, and one or more image generation and processing components 1168) configured to produce an ultrasound image from the beamformed signals (e.g., beamformed RF data).

The signal processor 1126 may be configured to process the received beamformed RF data in various ways, such as bandpass filtering, decimation, I and Q component separation, and harmonic signal separation. The signal processor 1126 may also perform additional signal enhancement such as speckle reduction, signal compounding, and noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, Doppler image data). For example, the system may include a B-mode signal path 1158 which couples the signals from the signal processor 1126 to a B-mode processor 1128 for producing B-mode image data.

The B-mode processor can employ amplitude detection for the imaging of structures in the body. The signals produced by the B-mode processor 1128 may be coupled to a scan converter 1130 and/or a multiplanar reformatter 1132. The scan converter 1130 may be configured to arrange the echo signals from the spatial relationship in which they were received to a desired image format. For instance, the scan converter 1130 may arrange the echo signal into a two dimensional (2D) sector-shaped format, or a pyramidal or otherwise shaped three dimensional (3D) format. The multiplanar reformatter 1132 can convert echoes which are received from points in a common plane in a volumetric region of the body into an ultrasonic image (e.g., a B-mode image) of that plane, for example as described in U.S. Pat. No. 6,443,896 (Detmer). The scan converter 1130 and multiplanar reformatter 1132 may be implemented as one or more processors in some embodiments.

A volume renderer 1134 may generate an image (also referred to as a projection, render, or rendering) of the 3D dataset as viewed from a given reference point, e.g., as described in U.S. Pat. No. 6,530,885 (Entrekin et al.). The volume renderer 1134 may be implemented as one or more processors in some embodiments. The volume renderer 1134 may generate a render, such as a positive render or a negative render, by any known or future known technique such as surface rendering and maximum intensity rendering.

In some embodiments, the system may include a Doppler signal path 1162 which couples the output from the signal processor 1126 to a Doppler processor 1160. The Doppler processor 1160 may be configured to estimate the Doppler shift and generate Doppler image data. The Doppler image data may include color data which is then overlaid with B-mode (i.e. grayscale) image data for display. The Doppler processor 1160 may be configured to filter out unwanted signals (i.e., noise or clutter associated with non-moving tissue), for example using a wall filter. The Doppler processor 1160 may be further configured to estimate velocity and power in accordance with known techniques. For example, the Doppler processor may include a Doppler estimator such as an auto-correlator, in which velocity (Doppler frequency, spectral Doppler) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. The velocity and/or power estimates may then be mapped to a desired range of display colors in accordance with a color map. The color data, also referred to as Doppler image data, may then be coupled to the scan converter 1130, where the Doppler image data may be converted to the desired image format and overlaid on the B-mode image of the tissue structure to form a color Doppler or a power Doppler image.

Outputs from the scan converter 1130, the multiplanar reformatter 1132, and/or the volume renderer 1134 may be coupled to an image processor 1136 for further enhancement, buffering and temporary storage before being displayed on an image display 1138. A graphics processor 1140 may generate graphic overlays for display with the images. These graphic overlays can contain, e.g., standard identifying information such as patient name, date and time of the image, imaging parameters, and the like. For these purposes the graphics processor 1140 may be configured to receive input from the user interface 1124, such as a typed patient name or other annotations (e.g., labels, bodymarkers). The user interface 1124 can also be coupled to the multiplanar reformatter 1132 for selection and control of a display of multiple multiplanar reformatted (MPR) images.

The system 1100 may include local memory 1142. Local memory 1142 may be implemented as any suitable non-transitory computer readable medium (e.g., flash drive, disk drive). Local memory 1142 may store data generated by the system 1100 including ultrasound images, executable instructions, imaging parameters, or any other information necessary for the operation of the system 1100. In some examples, local memory 1142 may include multiple memories, which may be the same or of different type. For example, local memory 1142 may include a dynamic random access memory (DRAM) and a flash memory.

As mentioned previously system 1100 includes user interface 1124. User interface 1124 may include display 1138 and control panel 1152. The display 1138 may include a display device implemented using a variety of known display technologies, such as LCD, LED, OLED, or plasma display technology. In some embodiments, display 1138 may comprise multiple displays. The control panel 1152 may be configured to receive user inputs (e.g., exam type, patient parameters). The control panel 1152 may include one or more hard controls (e.g., buttons, knobs, dials, encoders, mouse, trackball or others). In some embodiments, the control panel 1152 may additionally or alternatively include soft controls (e.g., GUI control elements or simply, GUI controls) provided on a touch sensitive display. In some embodiments, display 1138 may be a touch sensitive display that includes one or more soft controls of the control panel 1152.

In some embodiments, various components shown in Fig. 11 may be combined. For instance, the image processor 1136 and graphics processor 1140 may be implemented as a single processor. In some embodiments, various components shown in Fig. 11 may be implemented as separate components. For example, signal processor 1126 may be implemented as separate signal processors for each imaging mode (e.g., B-mode, Doppler). In another example, the image processor 1136 may be implemented as separate processors for different tasks and/or parallel processing of a same task. In some embodiments, one or more of the various processors shown in Fig. 11 may be implemented by general purpose processors and/or microprocessors configured to perform the specified tasks. In some examples, the processors may be configured by providing instructions for the tasks from a non-transitory computer readable medium (e.g., from local memory 1142). The instructions may then be executed by the processors. In some embodiments, one or more of the various processors may be implemented as application specific circuits. In some embodiments, one or more of the various processors (e.g., image processor 1136) may be implemented with one or more graphical processing units (GPU).

According to examples of the present disclosure, the system 1100 may be used to perform an ultrasound lung scan to acquire one or more ultrasound images of a lung of a subject, such as ultrasound image 700. For example ultrasound probe 1112 may acquire one or more ultrasound images. In some examples, the system 1100 may acquire a series of temporally spaced ultrasound images, such as those used to compute a motion map, such as motion map 904. In some examples, the user interface 1124 may receive user inputs, such as an indication that a pleural line should be detected in the acquired ultrasound image, and/or information relating to the subject that may alter a predetermined depth (e.g., age, weight).

In some examples, one or more of the processors of system 1100 may perform the portions of the technique described in blocks 402-414 of Fig. 4. In some examples, image processor 1136 may automatically select the first, second, and third ROIs, compute the lateral and axial projections, analyze the projections to determine the rib shadow region, location of a rib surface, and/or candidate pleural lines, compute a motion map, and determine a location of the pleural line. In some examples, graphics processor 1140 may generate the visual indication of the pleural line, errors (e.g., no pleural line determined), and/or warnings (e.g., presence of PTX). In some examples, the ultrasound image (e.g., ultrasound image 700), motion map (e.g., motion map 904), and/or visual indications may be provided on display 1138.

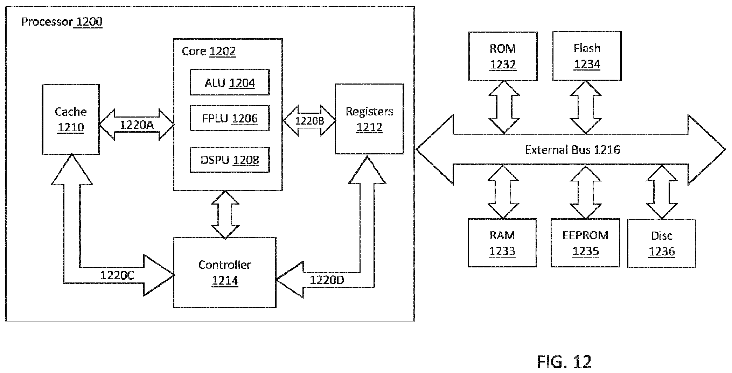

Fig. 12 is a block diagram illustrating an example processor 1200 according to principles of the present disclosure. Processor 1200 may be used to implement one or more processors and/or controllers described herein, for example, image processor 1136 shown in Fig. 11 and/or any other processor or controller shown in Fig. 11. Processor 1200 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 1200 may include one or more cores 1202. The core 1202 may include one or more arithmetic logic units (ALU) 1204. In some embodiments, the core 1202 may include a floating point logic unit (FPLU) 1206 and/or a digital signal processing unit (DSPU) 1208 in addition to or instead of the ALU 1204.

The processor 1200 may include one or more registers 1212 communicatively coupled to the core 1202. The registers 1212 may be implemented using dedicated logic gate circuits (e.g., flip-flops) and/or any memory technology. In some embodiments the registers 1212 may be implemented using static memory. The register may provide data, instructions and addresses to the core 1202.

In some embodiments, processor 1200 may include one or more levels of cache memory 1210 communicatively coupled to the core 1202. The cache memory 1210 may provide computer-readable instructions to the core 1202 for execution. The cache memory 1210 may provide data for processing by the core 1202. In some embodiments, the computer-readable instructions may have been provided to the cache memory 1210 by a local memory, for example, local memory attached to the external bus 1216. The cache memory 1210 may be implemented with any suitable cache memory type, for example, metal-oxide semiconductor (MOS) memory such as static random access memory (SRAM), dynamic random access memory (DRAM), and/or any other suitable memory technology.

The processor 1200 may include a controller 1214, which may control input to the processor 1200 from other processors and/or components included in a system (e.g., control panel 1152 and scan converter 1130 shown in Fig. 11) and/or outputs from the processor 1200 to other processors and/or components included in the system (e.g., display 1138 and volume renderer 1134 shown in Fig. 11). Controller 1214 may control the data paths in the ALU 1204, FPLU 1206 and/or DSPU 1208. Controller 1214 may be implemented as one or more state machines, data paths and/or dedicated control logic. The gates of controller 1214 may be implemented as standalone gates, FPGA, ASIC or any other suitable technology.

The registers 1212 and the cache memory 1210 may communicate with controller 1214 and core 1202 via internal connections 1220A, 1220B, 1220C and 1220D. Internal connections may implemented as a bus, multiplexor, crossbar switch, and/or any other suitable connection technology. Inputs and outputs for the processor 1200 may be provided via a bus 1216, which may include one or more conductive lines. The bus 1216 may be communicatively coupled to one or more components of processor 1200, for example the controller 1214, cache memory 1210, and/or register 1212. The bus 1216 may be coupled to one or more components of the system, such as display 1138 and control panel 1152 mentioned previously.

The bus 1216 may be coupled to one or more external memories. The external memories may include Read Only Memory (ROM) 1232. ROM 1232 may be a masked ROM, Electronically Programmable Read Only Memory (EPROM) or any other suitable technology. The external memory may include Random Access Memory (RAM) 1233. RAM 1233 may be a static RAM, battery backed up static RAM, Dynamic RAM (DRAM) or any other suitable technology. The external memory may include Electrically Erasable Programmable Read Only Memory (EEPROM) 1235. The external memory may include Flash memory 1234. The external memory may include a magnetic storage device such as disc 1236. In some embodiments, the external memories may be included in a system, such as ultrasound imaging system 1100 shown in Fig. 11, for example local memory 1142.

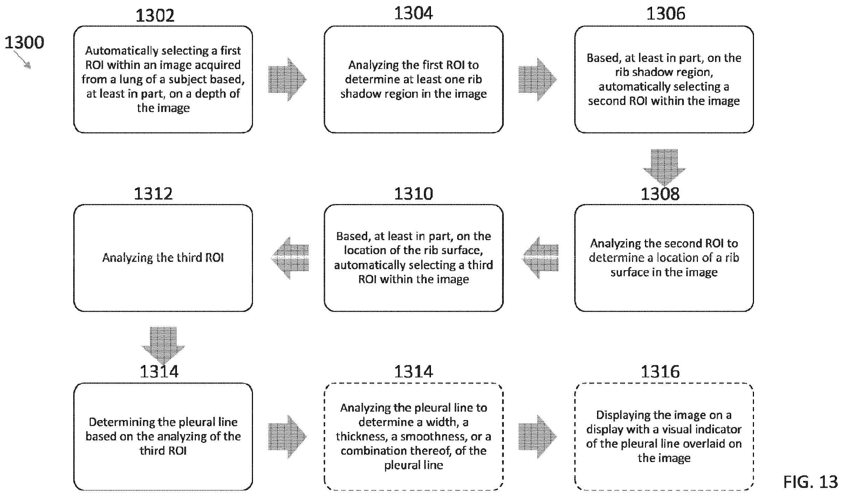

Fig. 13 is a flow chart of a method in accordance with examples of the present disclosure. In some examples, the method 1300 may be performed in whole or in part by an ultrasound imaging system, such as ultrasound imaging system 1100. The method 1300 may be a method for determining a pleural line in an image, such as image 700.

At block 1302, "automatically selecting a first ROI within an image acquired from a lung of a subject based, at least in part, on a depth of the image" may be performed. In some examples, the image may be an ultrasound image. In some examples, the image may have been acquired by an ultrasound probe, such as ultrasound probe 1114 of system 1100. In some examples, the first region of interest extends from a pre-determined depth to the depth (e.g., bottom) of the image. For example, the first ROI may extend from [(Depth)-(Pre-determined depth)] to [Depth] as shown in Fig. 6. In some examples, the first ROI may be selected by at least one processor, such as image processor 1136 of system 1100 and/or processor 1200. In some examples, first ROI may be based, at least in part, on a user input. For example, the user input may be used to select and/or adjust the pre-determined depth. The user input may indicate information related to the subject such as age of the subject, weight of the subject, or a combination thereof. In some examples, the user input may be received at a user interface, such as user interface 1124.

At block 1304, "analyzing the first ROI to determine at least one rib shadow region in the image" may be performed. In some examples, the analyzing may be performed by the at least one processor. In some examples, analyzing the first ROI may include computing, with at least one processor, a lateral projection for the first region of interest, for example, as described with reference to Figs. 4 and 5A.

At block 1306, "based, at least in part, on the rib shadow region, automatically selecting a second ROI within the image" may be performed. In some examples, the selecting may be performed by the at least one processor. In some examples, the second region of interest extends from a top of the image to the depth of the image and extends across a portion of a width of the image based on a comparison of lateral intensities of the lateral projection to a threshold value as shown in Figs. 6, 7B and 8A. In some examples, the width may be based on lateral intensities that are equal to and/or less than the threshold value. In some examples, low lateral intensities may indicate the rib shadow region in the image.

At block 1308, "analyzing the second ROI to determine a location of a rib surface in the image" may be performed. In some examples, the analyzing may be performed by the at least one processor. In some examples, analyzing the second ROI includes computing, with at least one processor, an axial projection for the second region of interest and detecting, with the at least one processor, a peak in the axial projection. In some examples, the peak is detected based, at least in part, on a comparison of a difference between a peak value and a neighboring value and a threshold value. For example, as described with reference to Fig. 4, Fig. 5B, and Figs. 8A and 8B.

At block 1310, "based, at least in part, on the location of the rib surface, automatically selecting a third ROI within the image" may be performed. In some examples, the selecting may be performed by the at least one processor. In some examples, the third region of interest extends from a first depth in the image corresponding to a location of the peak to a second depth greater than the first depth as shown in Figs. 6, 8A, and 9A.

At block 1312, "analyzing the third ROI" may be performed. In some examples, the analyzing may be performed by the at least one processor. In some examples, analyzing the third ROI may include computing, with at least one processor, an axial projection for the third region of interest, detecting, with the at least one processor, one or more peaks in the second axial projection, wherein individual ones of the one or more peaks correspond to a corresponding candidate pleural line, and computing, with the at least one processor, a motion map for the third region of interest. The motion map may be computed as described with reference to Figs. 4 and 9B. Optionally, analyzing the third ROI may further include determining a location of individual ones of the candidate pleural lines based, at least in part, on locations of corresponding ones of the one or more peaks and/or determining a local brightness of individual ones of the candidate pleural lines. The determining may be performed by the at least one processor in some examples.

At block 1314, "determining the pleural line based on the analyzing of the third ROI" may be performed. In some examples, the determining may be performed by the at least one processor. In some examples, determining the pleural line may include for individual ones of the corresponding candidate pleural lines: calculating an average motion above the candidate pleural line and an average motion below the candidate pleural line from the motion map and calculating a difference between the average motion above and the average motion below the candidate pleural line. Determining the pleural line may further include determining the candidate pleural line having a greatest difference between the average motion above and the average motion below and selecting the candidate pleural line having the greatest difference as the pleural line.

Optionally, method 1300 may further include "analyzing the pleural line to determine a width, a thickness, a smoothness, or a combination thereof, of the pleural line" at block 1316. In some examples, the analyzing may be performed by the at least one processor.

Optionally, method 1300 may further include, "displaying the image on a display with a visual indicator of the pleural line overlaid on the image" as indicated by block 1318. In some examples, the display may include display 1138. In some examples, the visual indicator may be generated by the at least one processor, such as graphics processor 1140.

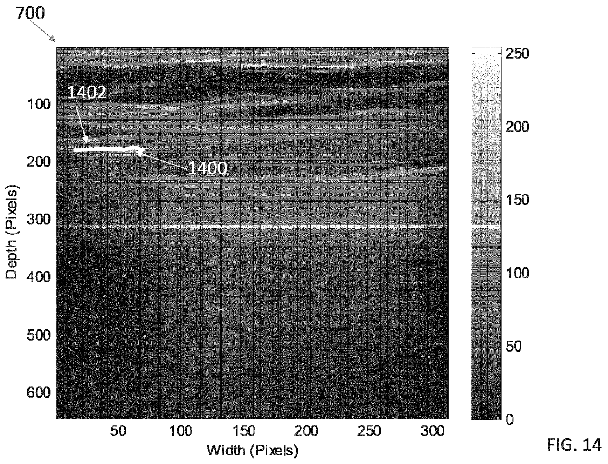

While the examples of the present disclosure have highlighted techniques for determining (e.g., identifying, detecting) a pleural line within an image, the techniques disclosed herein may be used to determine a rib and/or surface of a rib in the image. In these examples, blocks 1302-1308 of method 1300 may be performed. Optionally, in some examples, instead of showing a visual indicator of the pleural line (e.g., Fig. 10 and block 1316), a visual indicator of the rib and/or rib surface may be displayed overlaid on the image. Fig. 14 shows the ultrasound image shown in Fig. 7A with a visual indication of a rib surface in accordance with examples of the present disclosure. In image 700, the rib surface 1400 is highlighted by a curve 1402. If the pleural line could not be determined, an indication of such may be provided (e.g., error text or symbol). In some examples, visual indications of the rib surface and the pleural line may be provided on a display.

An automated system and method for detection and quantifying pleural-line using ultrasound imaging systems in emergency cases of acute respiratory diseases or thoracic diseases is disclosed herein. The system and method may be used in pre-hospital settings, initial evaluation in the emergency room, and/or follow-up after proper treatments. The systems and methods may be applicable to all ultrasound imaging systems, especially in point-of-care applications ranging from trauma to surgeon induced, can be used in a variety of settings including ambulance, ER, or critical care, or surgery situations. The systems and methods disclosed herein may improve automated detection and analysis of the pleural line without requiring access to RF data or the high computing demands of AI approaches in some applications.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "C#", "Java", "Python", and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments.
Accordingly, the specification and drawings are to be regarded in an illustrative manner and are not intended to limit the scope of the appended claims.

## Claims

1. A computer-implemented method for determining a pleural line (902, 1000) in an ultrasound image (700), the method comprising:
automatically selecting a first region of interest (ROI) (602, 704) within an ultrasound image (600, 700) acquired from a lung of a subject based, at least in part, on a depth of the image;
analyzing the first ROI to determine at least one rib shadow region (706) in the image; based, at least in part, on the rib shadow region, automatically selecting a second ROI (604, 800) within the image;
analyzing the second ROI to determine a location of a rib surface (808) in the image;
based, at least in part, on the location of the rib surface, automatically selecting a third ROI (606, 810) within the image;
analyzing the third ROI; and
determining the pleural line (902, 1000) based on the analyzing of the third ROI,
wherein analyzing the third ROI includes:
computing an axial projection for the third region of interest,
detecting one or more peaks in the axial projection, wherein individual ones of the one or more peaks correspond to a corresponding candidate pleural line, and
computing a motion map for the third region of interest from a series of ultrasound images which include the ultrasound image used to select the first, second and third ROI.

2. The method of claim 1, wherein the first ROI extends from a pre-determined depth to the depth of the image.

3. The method of claim 1, wherein analyzing the first ROI comprises computing, with at least one processor, a lateral projection for the first region of interest.

4. The method of claim 3, wherein the second ROI extends from a top of the image to the depth of the image and extends across a portion of a width of the image based on a comparison of lateral intensities of the lateral projection to a threshold value.

5. The method of claim 1, wherein analyzing the second ROI comprises:
computing, with at least one processor, an axial projection for the second region of interest; and
detecting, with the at least one processor, a peak in the axial projection.

6. The method of claim 5, wherein the third ROI extends from a first depth in the image corresponding to a location of the peak to a second depth greater than the first depth.

7. The method of claim 5, wherein the peak is detected based, at least in part, on a comparison of a difference between a peak value and a neighboring value and a threshold value.

8. The method of claim 1, wherein determining the pleural line comprises:
for individual ones of the corresponding candidate pleural lines:
calculating an average motion above the candidate pleural line and an average motion below the candidate pleural line from the motion map; and
calculating a difference between the average motion above and the average motion below the candidate pleural line;
determining the candidate pleural line having a greatest difference between the average motion above and the average motion below; and
selecting the candidate pleural line having the greatest difference as the pleural line.

9. The method of claim 1, further comprising determining a location of individual ones of the candidate pleural lines based, at least in part, on locations of corresponding ones of the one or more peaks.

10. The method of claim 1, further comprising determining a local brightness of individual ones of the candidate pleural lines.

11. The method of claim 1, further comprising:
displaying the image on a display with a visual indicator of the pleural line overlaid on the image.

12. An ultrasound imaging system (1100) configured to determine a pleural line (902, 1000) in an ultrasound image (700), the system comprising:
an ultrasound probe (1114) configured to acquire an ultrasound image (700) from a lung of a subject;
at least one processor (1136, 1140, 1200) configured to:
automatically select a first region of interest (ROI) within the ultrasound image based, at least in part, on a depth of the ultrasound image;
analyze the first ROI to determine at least one rib shadow region in the ultrasound image;
based, at least in part, on the rib shadow region, automatically select a second ROI within the ultrasound image;
analyze the second ROI to determine a location of a rib surface in the ultrasound image;
based, at least in part, on the location of the rib surface, automatically select a third ROI within the ultrasound image;
analyze the third ROI; and
determine the pleural line based on the analyzing of the third ROI; and
a display (1138) configured to display a visual indication of the pleural line overlaid on the ultrasound image;
wherein the analyzing the third ROI includes:
computing an axial projection for the third region of interest,
detecting one or more peaks in the axial projection, wherein individual ones of the one or more peaks correspond to a corresponding candidate pleural line, and
computing a motion map for the third region of interest from a series of ultrasound images which include the ultrasound image used to select the first, second and third ROI.

13. A computer program product comprising instructions which, when the program is executed by a computer, cause the computer to carry out the steps of the method of any of claims 1-12.

## Patentansprüche

1. Computerimplementiertes Verfahren zur Bestimmung einer Pleuralinie (902, 1000) in einem
Ultraschallbild (700), wobei das Verfahren umfasst:
automatisches Auswählen eines ersten Interessenbereichs (ROI) (602, 704) innerhalb eines Ultraschallbildes (600, 700), das von einer Lunge eines Probanden aufgenommen wurde, basierend mindestens teilweise auf einer Tiefe des Bildes;
Analysieren des ersten ROI, um mindestens einen Rippenschattenbereich (706) im Bild zu bestimmen; basierend mindestens teilweise auf dem Rippenschattenbereich, automatisches Auswählen eines zweiten ROI (604. 800) innerhalb des Bildes;
Analysieren des zweiten ROI zur Bestimmung der Stelle einer Rippenoberfläche (808) im Bild;
basierend mindestens teilweise auf der Stelle der Rippenoberfläche, automatisches Auswählen eines dritten ROI (606, 810) innerhalb des Bildes;
Analysieren des dritten ROI; und
Bestimmen der Pleuralinie (902, 1000) basierend auf der Analyse des dritten ROI,
wobei Analyse des dritten ROI einschließt:
Berechnen einer axialen Projektion für den dritten Interessensbereich,
Erfassen einer oder mehrerer Spitzen in der axialen Projektion, wobei einzelne der einen oder mehreren Spitzen einer entsprechenden Kandidaten-Pleuralinie entsprechen, und
Berechnen einer Bewegungskarte für den dritten Interessenbereich aus einer Reihe von Ultraschallbildern, die das Ultraschallbild einschließen, das zur Auswahl des ersten, zweiten und dritten ROI verwendet wurde.

2. Verfahren nach Anspruch 1, wobei sich der erste ROI von einer vorbestimmten Tiefe bis zur Tiefe des Bildes erstreckt.

3. Verfahren nach Anspruch 1, wobei Analyse des ersten ROI Berechnen einer lateralen Projektion für den ersten Interessenbereich mit mindestens einem Prozessor umfasst.

4. Verfahren nach Anspruch 3, wobei sich der zweite ROI von einer Oberseite des Bildes bis zur Tiefe des Bildes erstreckt und sich über einen Teil einer Breite des Bildes erstreckt, basierend auf einem Vergleich der lateralen Intensitäten der lateralen Projektion mit einem Schwellenwert.

5. Verfahren nach Anspruch 1, wobei Analyse des zweiten ROI umfasst:
Berechnen einer axialen Projektion für den zweiten Interessensbereich mit mindestens einem Prozessor; und Erfassen einer Spitze in der axialen Projektion mit dem mindestens einen Prozessor.

6. Verfahren nach Anspruch 5, wobei sich der dritte ROI von einer ersten Tiefe im Bild, die einer Stelle der Spitze entspricht, bis zu einer zweiten Tiefe erstreckt, die größer als die erste Tiefe ist.

7. Verfahren nach Anspruch 5, wobei die Spitze mindestens teilweise basierend auf einem Vergleich einer Differenz zwischen einem Spitzenwert und einem benachbarten Wert und einem Schwellenwert erfasst wird.

8. Verfahren nach Anspruch 1, wobei Bestimmung der Pleuralinie umfasst:
für einzelne der entsprechenden Kandidaten-Pleuralinien:
Berechnen einer durchschnittlichen Bewegung oberhalb der Kandidaten-Pleuralinie und einer durchschnittlichen Bewegung unterhalb der Kandidaten-Pleuralinie von der Bewegungskarte; und
Berechnen einer Differenz zwischen der durchschnittlichen Bewegung oberhalb und der durchschnittlichen Bewegung unterhalb der Kandidaten-Pleuralinie;
Bestimmen der Kandidaten-Pleuralinie, die den größten Unterschied zwischen der durchschnittlichen Bewegung oberhalb und der durchschnittlichen Bewegung unterhalb aufweist; und
Auswählen der Kandidaten-Pleuralinie, die den größten Unterschied aufweist, als Pleuralinie.

9. Verfahren nach Anspruch 1, weiter umfassend Bestimmen einer Stelle einzelner der Kandidaten-Pleuralinien basierend mindestens teilweise auf Stellen entsprechender der einen oder mehreren Spitzen.

10. Verfahren nach Anspruch 1, weiter umfassend Bestimmen einer lokalen Helligkeit einzelner der Kandidaten-Pleuralinien.

11. Verfahren nach Anspruch 1, weiter umfassend:
Anzeigen des Bildes auf einer Anzeige, wobei ein visueller Indikator für die Pleuralinie über das Bild gelegt wird.

12. Ultraschallbildgebungssystem (1100), das zur Bestimmung einer Pleuralinie (902, 1000) in einem Ultraschallbild (700) konfiguriert ist, wobei das System umfasst:
eine Ultraschallsonde (1114), die zum Aufnehmen eines Ultraschallbildes (700) von einer Lunge eines Probanden konfiguriert ist;
mindestens einen Prozessor (1136, 1140, 1200), der konfiguriert ist zum:
automatischen Auswählen eines ersten Interessenbereichs (ROI) innerhalb des Ultraschallbildes basierend mindestens teilweise auf einer Tiefe des Ultraschallbildes;
Analysieren des ersten ROI, um mindestens einen Rippenschattenbereich im Ultraschallbild zu bestimmen;
basierend mindestens teilweise auf dem Rippenschattenbereich, automatischen Auswählen eines zweiten ROI innerhalb des Ultraschallbildes;
Analysieren des zweiten ROI, um eine Stelle einer Rippenoberfläche im Ultraschallbild zu bestimmen;
basierend mindestens teilweise auf der Stelle der Rippenoberfläche, automatischen Auswählen eines dritten ROI innerhalb des Ultraschallbildes;
Analysieren des dritten ROI; und
Bestimmen der Pleuralinie anhand der Analyse des dritten ROI; und
eine Anzeige (1138), die zum Anzeigen einer visuellen Angabe der Pleuralinie überlagert auf dem Ultraschallbild konfiguriert ist;
wobei die Analyse des dritten ROI einschließt:
Berechnen einer axialen Projektion für den dritten Interessensbereich,
Erfassen einer oder mehrerer Spitzen in der axialen Projektion, wobei einzelne der einen oder mehreren Spitzen einer entsprechenden Kandidaten-Pleuralinie entsprechen, und
Berechnen einer Bewegungskarte für den dritten Interessenbereich aus einer Reihe von Ultraschallbildern, die das Ultraschallbild einschließen, das zur Auswahl des ersten, zweiten und dritten ROI verwendet wurde.

13. Computerprogrammprodukt, das Anweisungen umfasst, die, wenn das Programm von einem Computer ausgeführt wird, den Computer veranlassen, die Schritte des Verfahrens nach einem der Ansprüche 1-12 durchzuführen.

## Revendications

1. Procédé mis en œuvre par ordinateur pour déterminer une ligne pleurale (902. 1000) dans une
image ultrasonore (700), le procédé comprenant :
la sélection automatique d'une première région d'intérêt (ROI) (602, 704) dans une image ultrasonore (600, 700) acquise à partir d'un poumon d'un sujet sur la base, au moins en partie, de la profondeur de l'image ;
l'analyse de la première ROI pour déterminer au moins une région d'ombre de côte (706) dans l'image ; sur la base, au moins en partie, de la région d'ombre de côte, la sélection automatique d'une deuxième ROI (604, 800) dans l'image ;
l'analyse de la deuxième ROI pour déterminer l'emplacement d'une surface de côte (808) dans l'image ;
sur la base, au moins en partie, de l'emplacement de la surface de côte, la sélection automatique d'une troisième ROI (606, 810) dans l'image ;
l'analyse de la troisième ROI ; et
la détermination de la ligne pleurale (902, 1000) sur la base de l'analyse de la troisième ROI.
dans lequel l'analyse de la troisième ROI inclut :
le calcul d'une projection axiale pour la troisième région d'intérêt.
la détection d'un ou de plusieurs pics dans la projection axiale, dans lequel des pics individuels de un ou plusieurs pics correspondent à une ligne pleurale candidate correspondante, et
le calcul d'une carte de mouvement pour la troisième région d'intérêt à partir d'une série d'images ultrasonores qui incluent l'image ultrasonore utilisée pour sélectionner la première, la deuxième et la troisième ROI.

2. Procédé selon la revendication 1, dans lequel la première ROI s'étend depuis une profondeur prédéterminée jusqu'à la profondeur de l'image.

3. Procédé selon la revendication 1, dans lequel l'analyse de la première ROI comprend le calcul, avec au moins un processeur, d'une projection latérale pour la première région d'intérêt.

4. Procédé selon la revendication 3, dans lequel la deuxième ROI s'étend du haut de l'image jusqu'à la profondeur de l'image et s'étend sur une partie de la largeur de l'image sur la base d'une comparaison d'intensités latérales de la projection latérale à une valeur seuil.

5. Procédé selon la revendication 1, dans lequel l'analyse de la deuxième ROI comprend :
le calcul, avec au moins un processeur, d'une projection axiale pour la deuxième région d'intérêt ; et la détection, avec le au moins un processeur, d'un pic dans la projection axiale.

6. Procédé selon la revendication 5, dans lequel la troisième ROI s'étend depuis une première profondeur dans l'image correspondant à un emplacement du pic jusqu'à une seconde profondeur plus importante que la première profondeur.

7. Procédé selon la revendication 5, dans lequel le pic est détecté sur la base, au moins en partie, d'une comparaison d'une différence entre une valeur de pic et une valeur voisine et une valeur seuil.

8. Procédé selon la revendication 1, dans lequel la détermination de la ligne pleurale comprend :
pour des lignes pleurales individuelles des lignes pleurales candidates correspondantes :
le calcul d'un mouvement moyen au-dessus de la ligne pleurale candidate et d'un mouvement moyen en dessous de la ligne pleurale candidate à partir de la carte de mouvement ; et
le calcul de la différence entre le mouvement moyen au-dessus de la ligne pleurale candidate et le mouvement moyen en dessous de la ligne pleurale candidate ;
la détermination de la ligne pleurale candidate présentant la plus grande différence entre le mouvement moyen au-dessus de la ligne plurale candidate et le mouvement moyen en-dessous de celle-ci ; et
la sélection de la ligne pleurale candidate présentant la plus grande différence comme ligne pleurale.

9. Procédé selon la revendication 1 comprenant en outre la détermination de l'emplacement de lignes pleurales individuelles des lignes pleurales candidates sur la base, au moins en partie, de l'emplacement de pics correspondants des un ou plusieurs pics.

10. Procédé selon la revendication 1 comprenant en outre la détermination d'une luminosité locale de lignes pleurales individuelles des lignes pleurales candidates.

11. Procédé selon la revendication 1 comprenant en outre :
l'affichage de l'image sur un écran avec un indicateur visuel de la ligne pleurale superposée à l'image.

12. Système d'imagerie par ultrasons (1100) configuré pour déterminer une ligne pleurale (902, 1000) dans une image ultrasonore (700), le système comprenant :
une sonde à ultrasons (1114) configurée pour acquérir une image ultrasonore (700) d'un poumon d'un sujet ;
au moins un processeur (1136, 1140, 1200) configuré pour :
sélectionner automatiquement une première région d'intérêt (ROI) dans l'image ultrasonore sur la base, au moins en partie, de la profondeur de l'image ultrasonore ;
analyser la première ROI pour déterminer au moins une zone d'ombre de côte dans l'image ultrasonore ;
sur la base, au moins en partie, de la région d'ombre de côte, sélectionner automatiquement une deuxième ROI dans l'image ultrasonore ;
analyser la deuxième ROI pour déterminer l'emplacement d'une surface de côte sur l'image ultrasonore ;
sur la base, au moins en partie, de l'emplacement de la surface de côte, sélectionner automatiquement une troisième ROI dans l'image ultrasonore ;
analyser le troisième ROI ; et
déterminer la ligne pleurale sur la base de l'analyse de la troisième ROI ; et
un écran (1138) configuré pour afficher une indication visuelle de la ligne pleurale superposée à l'image ultrasonore ;
dans lequel l'analyse de la troisième ROI inclut :
le calcul d'une projection axiale pour la troisième région d'intérêt,
la détection d'un ou de plusieurs pics dans la projection axiale, dans lequel des pics individuels des un ou plusieurs pics correspondent à une ligne pleurale candidate correspondante, et
le calcul d'une carte de mouvement pour la troisième région d'intérêt à partir d'une série d'images ultrasonores qui incluent l'image ultrasonore utilisée pour sélectionner la première, la deuxième et la troisième ROI.

13. Produit de programme informatique comprenant des instructions qui, lorsque le programme est exécuté par un ordinateur, amènent l'ordinateur à exécuter les étapes du procédé selon l'une quelconque des revendications 1-12.
